# EUROPEAN PATENT APPLICATION

(11) **EP 1 911 849 A1**
(43) Date of publication of application: **16.04.2008**
(21) Application number: 06781774.2
(22) Date of filing: 27.07.2006
(51) Int. Cl.: C12P 7/22, A23L 1/30, A61K 31/085, A61K 35/74, A61P 15/12

(54) **PROPHYLACTIC/AMELIORATING AGENT FOR MENOPAUSAL DISORDER AND FUNCTIONAL BEVERAGE/FOOD**

(30) Priority: 29.07.2005 JP 2005221984
(71) Applicant: Calpis Co., Ltd., Shibuya-ku, Tokyo 150-0022 (JP)
(72) Inventor: HATTORI, Masao, Institute of Natural Medicine, Toyama-shi, Toyoma 930-0194 (JP); NAKAMURA, Norio, Fac. of Pharmaceutical Sciences, Kyotanabe-shi (JP); YOSHIMURA, Chiaki, Sagamihara-shi, Kanagawa 229-0006 (JP); MASUYAMA, Akihiro, Sagamihara-shi, Kanagawa 229-0006 (JP); TAKANO, Toshiaki, Sagamihara-shi, Kanagawa 229-0006 (JP)
(74) Representative: Jappy, John William Graham
(86) International application number: PCT/JP2006/314863
(87) International publication number: WO 2007/013547

(57) **Abstract**

Disclosed is a method for producing an estrogen-like active intermediate (e.g., secoisolariciresinol) by culturing a lignan (e.g., a lignan derived from Eucommia ulmoides) together with a bacterium Lactobacillus acidophilus. Also disclosed a prophylactic/ameliorating agent for menopausal disorder and a functional beverage/food for preventing/ameliorating menopausal disorder comprising a lignan cultured together with a bacterium Lactobacillus acidophilus as an active ingredient.

## Description

### Technical Field

The present invention relates to a prophylactic/ameliorating agent for menopausal disorder and a functional beverage/food for preventing/ameliorating various symptoms associated with menopause.

### Background Art

Menopausal disorder is a physical and psychological indefinite complaint caused by decrease in secretion of female hormone (estrogen) from ovary around menopause. Thereafter various symptoms are presented with age, including aging of skin, hyperlipemia, osteoporosis and decrease in brain function.

It was thought that a hormone replacement treatment (HRT) by administering female hormone was effective for these symptoms. However, in the intermediate report at a time of 5 year-use published in WHI (Woman's Health Initiative) in July, 2002, there was reported a risk of an increase in a developmental rate of breast cancer resulting from administration of estrogen. Therefore, a safer menopausal therapy has now been desired.

It is known that Japanese are less suffered from menopausal disorders than westerners. There is a report that one reason of it relates to the ingestion of soybeans and fermented foods of soybeans. Namely, it is thought that isoflavone contained in soybeans has an estrogen-like activity. It is also known that ingestion of various plants other than soybean shows an estrogen-like activity. Plants that show an estrogen-like activity via indigestion include sesame, linseed, du zhong. In fact, supplements containing these plants as raw materials are commercially available. Further, Milder et al. (Ivon E. J. Milder et al., Br. J. Nutr., 93, 393-402 (2005); Non-patent Document 1) reported the contents of 4 types of lignans (lariciresinol, pinoresinol, secoisolariciresinol and metairesinol) in food products derived from plants.

It has been reported that ingestion of du zhong that contains a lignan having an estrogen-like activity showed improvement in various symptoms of menopausal disorder. In a Chinese comprehensive dictionary of medicines and a mainland Chinese medical dictionary, there is a description "there are potencies to cover for liver and kidney, strengthen muscle and bone, and ease up fetal presentation; to cure an stinging pain of waist and back, weakness of foot and knee (paralysis of knee), residual urine, abnormal bleed of woman, premature abortion, high blood pressure and the like." Further, antihyperlipidemic function of lignans is specifically shown in JP B 3435415 (Patent Document 1).

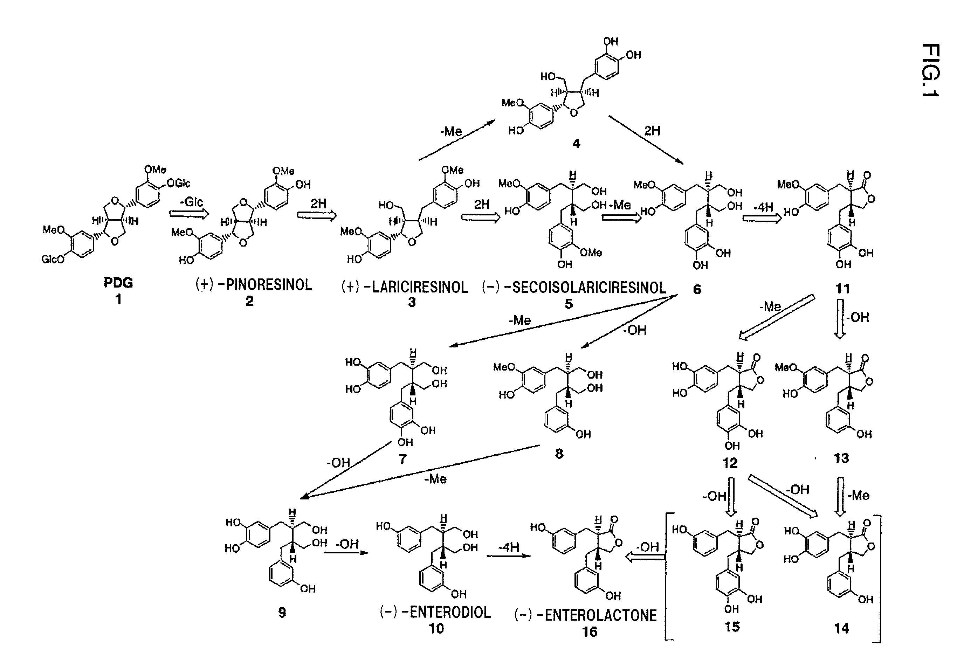

It is thought that a type of lignan, pinoresinol diglycoside, is deglycosylated by an action of intestinal bacterium into pinoresinol, lariciresinol and secoisolariciresinol, and finally metabolized into enterodiol and enterolactone to exhibit an estrogen-like activity (Fig. 1) (Li-Hua Xie, et al., Chem. Pharm. Bull. 51 (5)508-515 (2003); Non-patent Document 2). However, there have not been elucidated to date what bacteria or bacterial group have an activity to metabolize lignan, and which component of various metabolic intermediates has an estrogen-like activity.

The degree of effects achieved by ingestion of sesame, flaxseed (linseed), Eucommia ulmoides or supplement containing them as a raw material may vary depending on individuals. One of the reasons is thought to be the fact that each person has a different intestinal bacterium flora Namely, when a person ingests a natural medicine, active ingredients contained in the natural medicine are often metabolized/activated by intestinal bacterium to exhibit a certain effect. In some cases, however, the metabolism/activation of active ingredients do not proceed due to the type or the number of intestinal bacterium and no effect can be found.

Hence, in order to obtain an estrogen-like activity for aiming at preventing/ameliorating menopausal disorders, metabolizing/activating a lignan before ingestion would be highly effective than ingesting a lignan contained in plants as it is.

On the other hand, it is not easy to selectively obtain an estrogen-like active intermediate having a greater activity by chemical conversion of a lignan. Therefore, to obtain preventing/ameliorating effect of menopausal disorders, it is believed to be advantageous to develop a method for previously metabolizing/activating a lignan by biological means.

Reference documents referred to in the present specification are as follows. The contents described in these documents are incorporated herein by reference in its entirety. It is not admitted that any one of these documents is a prior art with respect to the present invention.
[Patent Document 1]: Japanese Patent Publication No. 3435415 [Non-patent Document 1]: Ivon E. J. Milder et al., Br. J. Nutr., 93, 393-402 (2005)
[Non-patent Document 2]: Li-Hua Xie, et al., Chem. Pharm. Bull. 51 (5)508-515 (2003)

### Disclosure of the Invention

The object of the present invention is to provide a useful medicament and functional beverage/food for preventing/ameliorating menopausal disorders.

The present inventors have found that bacterium Lactobacillus acidophilus metabolizes a lignan to produce an estrogen-like active intermediates, and completed the present invention. The present invention provides a method for producing an estrogen-like active intermediate comprising culturing a lignan together with Lactobacillus acidophilus. Preferably, the lignan is pinoresinol or medioresinol. Also preferably the lignan is a lignan derived from Eucommia ulmoides. Also preferably, the estrogen-like active intermediate is secoisolariciresinol.

In another aspect, the present invention provides an agent for prophylactic/ameliorating menopausal disorder and a functional beverage/food for preventing/ameliorating menopausal disorder comprising as an active ingredient a lignan cultured together with Lactobacillus acidophilus.

Menopausal disorders can be prevented or ameliorated by ingesting a lignan cultured together with Lactobacillus acidophilus or by ingesting a lignan and Lactobacillus acidophilus simultaneously. The prophylactic/ameliorating agent for menopausal disorder and a functional beverage/food comprising the same can be safely orally-ingested because it contains as an active ingredient a lignan whose safety has been confirmed for an oral ingestion.

### Brief Description of the Drawings

FIG. 1 shows a supposed metabolic pathway of a type of lignan, pinoresinol diglycoside, by intestinal bacteria.

### Preferred Embodiments of the Invention

Hereinafter, the present invention will be described further in detail.
The prophylactic/ameliorating agent for menopausal disorder of the present invention comprises as an active ingredient a lignan metabolized/activated by culturing a lignan together with Lactobacillus acidophilus, which shows a greater activity than a lignan ingested before metabolized/activated. The lignan includes, for example, Eucommia ulmoides lignan, flaxseed(linseed) lignan, sesame lignan and the like. Preferably, the lignan is Eucommia ulmoides lignan. Lignan is first converted from lignan glycoside to aglycone by Lactobacillus acidophilus. Then pinoresinol and medioresinol are metabolized/activated by Lactobacillus acidophilus and converted into various estrogen-like active intermediates. As used herein, the estrogen-like active intermediate includes both a substance itself having an estrogen-like activity and a substance which will be metabolized in a living body upon ingestion and converted to a substance having an estrogen-like activity. The estrogen-like active intermediates include lariciresinol and secoisolariciresinol and the like. In the present invention, the estrogen-like active intermediate is preferably secoisolariciresinol.

As shown in Examples described below, it has now been found that a lignan is metabolized by Lactobacillus acidophilus to generate an estrogen-like active intermediate, and the estrogen-like active intermediate indeed has a binding ability to an estrogen receptor.

The strain of Lactobacillus acidophilus is not particularly limited, but include, for example, a Lactobacillus acidophilus CL-0062 (Independence Administrative Legal Entity; Natural Advanced Industrial Science and Technology, International Patent Organism Depositary, deposit number FERM BP-4980, deposit date: March 4' 1994), and a Lactobacillus acidophilus L92 (Independence Administrative Legal Entity; Natural Advanced Industrial Science and Technology, International Patent Organism Depositary, deposit number FERM BP-4981, deposit date: March 4 1994).

The Lactobacillus acidophilus CL-0062 has the following mycological properties.
1. Morphological property
   1) shape of cell: bacillus, 2) mobility: none, 3) presence of spore: none, 4) Gram staining: positive.
2. Physiological property
   1) catalase: negative, 2) indole generation: negative, 3) reduction of nitrate: negative, 4) behavior to oxygen: facultative anaerobic bacterium, 5) growth at 15°C: none, 6) DL(-) lactic acid is produced from glucose by homolactic fermentation without gas production. 7) degradability of various hydrocarbons: glucose; +, lactose; +, mannose; +, fructose; +, galactose; +, sucrose; +, arabinose; -, maltose; +, xylose; -, rhamnose; -, cellbiose; +, trehalose; +, melbiose; +, raffinose; +, mannitol; -, sorbitol; -, esclin; +, salicin; +, N-acetylglycosamine; +, amygdalin; +, gentiobiose; +, melecitose; -, dextorin; +, starch;-.

The Lactobacillus acidophilus L92 has the following mycological properties.
1. Morphological property
   1) shape of cell: bacillus, 2) mobility: none, 3) presence of spore: none, 4) Gram staining: positive.
2. Physiological property
   1) catalase: negative, 2) indole generation: negative, 3) reduction of nitrate: negative, 4) behavior to oxygen: facultative anaerobic bacterium, 5) growth at 15°C: none, 6) DL(-) lactic acid is produced from glucose by homolactic fermentation without gas production. 7) degradability of various hydrocarbons: glucose; +, lactose; +, mannose; +, fructose; +, galactose; +, sucrose; +, arabinose; -, maltose; +, xylose; -, rhamnose; -, cellbiose; +, trehalose; +, melbiose; -, raffinose; +, mannitol; -, sorbitol; -, esclin; +, salicin; +, N-acetylglycosamine; +, amygdalin; +, gentiobiose; +, melecitose; -, dextorin; -, starch;-.

A lignan may be easily obtained from sesame, flaxseed(linseed), Eucommia ulmoides or the like. A particularly preferable lignan is a Eucommia ulmoides lignan. An extract from bark or leaf of Eucommia ulmoides, optionally processed through fractionation, concentration, purification, can be used in the present invention. In addition, the extract or its concentrate can be further processed into powder by freeze drying, spray drying or the like.

To culture a lignan together with Lactobacillus acidophilus, bacterial cells of Lactobacillus acidophilus are inoculated in a culture medium containing a lignan, and cultured generally by standing still or stirring culture, for example, at 25°C to 45°C, preferably 30°C to 45°C. For example, a lignan can be added in the form of MeOH solution at a final concentration of 0.01 to 10 mM. It is preferable to use a starter preparation of Lactobacillus acidophilus cells which have been cultured in MRS medium, GAM medium, milk or the like at 37°C for 20 to 24 hours. The number of initial cells is preferably about 10⁵ to 10⁹ cells/ml. The cultivation time is 3 to 168 hours, preferably 12 to 96 hours. The culture can be stopped at the time when an acid degree of lactic acid becomes 1.5 or more. Aerobic/anaerobic conditions can be conventionally selected according to the type of strain to be used.

For the lignan-metabolized intermediate obtained by culturing a lignan together with Lactobacillus acidophilus to show an estrogen-like activity, the metabolic intermediate must bind to an estrogen receptor. A binding ability to an estrogen receptor can be examined, for example, by evaluating a proliferation activity of MCF-7 cells, or by conducting an estrogen receptor assay using yeast.

Further, to examine an action of a lignan-metabolized intermediate in living body, a lignan cultured together with Lactobacillus acidophilus is orally administered to a menopausal model animal whose ovary has been removed, and a bone density or blood cholesterol of the animal can be measured, which is indicative of the effect of the intermediate.

The prophylactic/ameliorating agent for menopausal disorder of the present invention can be produced by formulating a lignan cultured together with Lactobacillus acidophilus by a method known to those of skilled in the art. For example, a lignan cultured together with lactic acid bacteria can be formulated by blending in a suitable combination with a pharmaceutically acceptable carrier or a medium, for example, a sterilized water, physiological saline, plant oil, emulsifier, suspension agent, surfactant, stabilizer, flavoring compound, diluting agent, vehicle, antiseptic agent, binding agent and the like. For oral administration, it can be formulated as a tablet, pill, sugarcoated agent, capsule, liquid, gel, syrup, slurry, suspension and the like. The prophylactic/ameliorating agent for menopausal disorder of the present invention may contain another component known to have an action for a preventing/ameliorating menopausal disorder in addition to a lignan cultured together with lactic acid bacteria as an active ingredient.

The oral ingestion dose of the prophylactic/ameliorating agent for menopausal disorder of the present invention can be conventionally selected to obtain a desired effect, depending on the administration period, the continuity thereof and the like. The dosage may vary depending on age, weight, symptom, curing effect, administration route, treating hour and the like. Generally, it is in a range of 1 mg to 1000 mg for an adult per dose, and can be orally administered at one to several times per day. Notably, since a lignan and lactic acid bacteria are eatable, there is no limitation of the dosage from a safety point of view.

The prophylactic/ameliorating agent for menopausal disorder of the present invention may be administered after various symptoms associated with menopause are developed, or may be administered continuously every day or intermittently during a period of preventing such symptoms.

In another aspect of the present invention, a functional beverage/food comprising a lignan cultured together with lactic acid bacteria is provided. In the functional beverage/food of the present invention, a compounding ratio of the lignan cultured together with lactic acid bacteria can be conventionally selected according to the form of the functional beverage/food, its ingestion continuity, or the like, and is not particularly limited.

The above-described functional beverage/food may be provided in the form of, for example, fermented dairy products such as yogurt and lactic acid bacteria beverage; various processed beverages/foods that fermented milk whey or its concentrate is compounded, tee beverage, dry powder, tablet, capsules, granular agent and the like.

The above-described functional beverage/food may be combined with additives such as sugars, protein, lipid, vitamin, mineral, flavor or a mixture thereof. Further, it may contain an extract of a plant lignan.

The functional beverage/food of the present invention may be provided as a food or the like for a specified health use, which is a food and a beverage containing the prophylactic/ameliorating agent for menopausal disorder of the present invention, and is designated for use in prevention or amelioration of various symptoms associated with menopause.

The ingestion amount and ingestion period of the functional beverage/food of the present invention are not particularly limited. For example, it can be ingested continuously or intermittently before a situation necessary for an improving effect on each symptom of menopausal disorders occurs, and continuously or intermittently after such situation occurred.

The contents of all patents and reference documents explicitly referred to in the present specification are incorporated herein by reference in its entirety. Further, the contents described in the specification and drawings of Japanese Patent Application No. 2005-221984 on the basis for claim of priority of the present application are incorporated herein by reference in its entirety.

### Examples

Hereinafter, the present invention will be described in more detail with reference to Examples, which does not limit the scope of the present invention.

### Example 1. Lignan-metabolizing ability of Lactobacillus acidophilus

Lignan-metabolizing abilities of various types of microorganisms were evaluated. The various microorganisms shown in the following Tables 1 to 3 were pre-cultured in MRS medium or GAM medium at 37°C for 20 to 24 hours, and inoculated at 1 to 3% to 10 ml of MRS medium or GAM medium. Du zhong lignan (pinoresinol and medioresinol) was added at a final concentration of 0.5 mM in the medium, and continued to culture at 37°C. Samples were taken at regular intervals and assayed by HPLC to monitor the conversion of lignan to secoisolariciresinol. The results are shown in Tables 1 to 3, where o indicates that the lignan was converted in 50% or more, and x indicates that most of the lignan was not converted.

**Table 1**

| | pinoresinol to secoisolariciresinol |
|---|---|
| *Lactobacillus helveticus* | x |
| *Lactobacillus acidophilus* | ○ |
| *Lactobacillus gasseri* | x |
| *Lactobacillus paracasei* | x |
| *Lactobacillus casei* | x |
| *Lactobacillus plantarum* | x |
| *Lactobacillus fermentum* | x |
| *Lactobacillus reuteri* | x |
| *Lactobacillus lactis* | x |
| *Enterococcus faecalis* | x |
| *Enterococcus faecium* | x |
| *Bacillus subtilis* | x |
| *Bacillus coagulans* | x |
| *Bifidobacterium* | x |

**Table 2**

| | pinoresinol to secoisolariciresinol |
|---|---|
| *Lactobacillus acidophilus* (1) | ○ |
| *Lactobacillus acidophilus* (2) | ○ |
| *Lactobacillus acidophilus* (3) | ○ |
| *Lactobacillus acidophilus* (4) | ○ |
| *Lactobacillus acidophilus* (5) | ○ |
| *Lactobacillus acidophilus* (6) | ○ |
| *Lactobacillus acidophilus* (7) | ○ |
| *Lactobacillus acidophilus* (8) | ○ |
| *Lactobacillus acidophilus* (9) | ○ |
| *Lactobacillus amylovorus* | x |
| *Lactobacillus crispatus* | x |
| *Lactobacillus gallinarum* | x |
| *Lactobacillus johosonii* | x |

**Table 3**

| | medioresinol to secoisolariciresinol |
|---|---|
| *Lactobacillus acidophilus* (1) | ○ |
| *Lactobacillus acidophilus* (4) | ○ |

Tables 1 to 3 clearly show that Lactobacillus acidophilus is capable of producing secoisolariciresinol from pinoresinol. Further, it was shown that at least the strains of Lactobacillus acidophilus tested here was capable of producing secoisolariciresinol from medioresinol. These results demonstrate that Lactobacillus acidophilus has an ability of metabolizing/activating a lignan to convert it to an estrogen-like active intermediate.

### Example 2. Binding of lignan and its metabolic product to estrogen receptor

One millimolar of a test sample (pinoresinol diglycoside, pinoresinol and secoisolariciresinol) and 0.5 nM of tritium-labeled estradiol were reacted in 10 mM of tris buffer at pH 7.4 (with 0.1% BSA, 10% Glycerol, 1 mM DTT) containing a human estrogen receptor α or β at 25°C for 2 hours. After completion of the reaction, the receptor was collected and washed to measure the amount of the tritium-labeled estradiol bound to the receptor according to a conventional method. The binding level of [³H] Estradiol in the presence of a lignan or a metabolic intermediate was calculated with the binding of 0.5 nM of [³H] Estradiol to the receptor as 100%, which was subtracted from 100 to define a binding inhibition rate. The results are shown in the following table.

**Table 4**

| binding inhibition rate to estrogen receptor α | |
|---|---|
| | inhibition rate (%) |
| pinoresinol diglycoside | 4 |
| pinoresinol | 23 |
| secoisolariciresinol | 43 |

| binding inhibition rate to estrogen receptor β | |
|---|---|
| | inhibition rate (%) |
| pinoresinol diglycoside | 5 |
| pinoresinol | 30 |
| secoisolariciresinol | 52 |

As described above, a lignan and its metabolic product were shown to have a binding ability to an estrogen receptor. Further, it was shown that the binding ability to an estrogen receptor became stronger with being metabolized from pinoresinol diglycoside through pinoresinol to secoisolariciresinol in Lactobacillus acidophilus. The binding inhibition rate of secoisolariciresinol to an estrogen receptor was ten times higher than that of pinoresinol diglycoside. From these results, it is expected that a lignan is metabolized by Lactobacillus acidophilus and exhibit various actions via expression of target genes to achieve prevention/amelioration of menopausal disorders.

### Industrial Applicability

The present invention is useful for prevention/amelioration of menopausal disorders.

## Claims

1. A method for producing an estrogen-like active intermediate comprising culturing a lignan together with Lactobacillus acidophilus.

2. The method according to claim 1, wherein the lignan is pinoresinol or medioresinol.

3. The method according to claim 1, wherein the lignan is a lignan derived from Eucommia ulmoides.

4. The method according to any one of claims 1 to 3, wherein the estrogen-like active intermediate is secoisolariciresinol.

5. A prophylactic/ameliorating agent for menopausal disorder comprising as an active ingredient a lignan cultured together with Lactobacillus acidophilus.

6. A functional beverage/food for preventing/ameliorating menopausal disorder comprising as an active ingredient a lignan cultured together with Lactobacillus acidophilus.
